# EUROPÄISCHE PATENTSCHRIFT

(11) **EP 3 585 785 B1**
(45) Veröffentlichungstag und Bekanntmachung des Hinweises auf die Patenterteilung: **27.01.2021**
(21) Anmeldenummer: 18706467.0
(22) Anmeldetag: 15.02.2018
(51) Int. Cl.: C07D 409/12

(54) **VERFAHREN ZUR HERSTELLUNG VON METHYL 4-[(4,5-DIHYDRO-3-METHOXY-4-METHYL-5-OXO-1H-1,2,4-TRIAZOL-1-YL)CARBONYL)SULFAMOYL]-5-METHYLTHIOPHEN-3-CARBOXYLAT**
PROCESS FOR THE PREPARATION OF METHYL 4-[(4,5-DIHYDRO-3-METHOXY-4-METHYL-5-OXO-1H-1,2,4-TRIAZOL-1-YL)CARBONYL)SULFAMOYL]-5-METHYLTHIOPHENE-3-CARBOXYLATE
ROCÉDÉ POUR LA PRODUCTION DE 4-[(4,5-DIHYDRO-3-METHOXY-4-METHYL-5-OXO-1H-1,2,4-TRIAZOL-1-YL)CARBONYL)SULFAMOYL]-5-METHYLTHIOPHENE-3-CARBOXYLATE

(30) Priorität: 23.02.2017 EP 17157615
(43) Veröffentlichungstag der Anmeldung: 01.01.2020
(73) Patentinhaber: Bayer CropScience Aktiengesellschaft, 40789 Monheim am Rhein (DE); Bayer Aktiengesellschaft, 51373 Leverkusen (DE)
(72) Erfinder: PAZENOK, Sergii, 42699 Solingen (DE); FUNKE, Christian, 42799 Leichlingen (DE); MÜHLTHAU, Friedrich, August, 65779 Kelkheim-Fischbach (DE); FORD, Mark, James, 65207 Wiesbaden-Breckenheim (DE); NEEFF, Arnd, 51399 Burscheid (DE); SCHIFFER, Klaus-Ulrich, 42369 Wuppertal (DE)
(74) Vertreter: BIP Patents
(86) Internationale Anmeldenummer: PCT/EP2018/053771
(87) Internationale Veröffentlichungsnummer: WO 2018/153767

(56) Entgegenhaltungen:
- DE-A1- 19 933 260
- DE-A1-102004 063 192

## Beschreibung

Die Erfindung betrifft ein verbessertes Verfahren zur Herstellung von Methyl 4-[(4,5-dihydro-3-methoxy-4-methyl-5-oxo-1*H*-1,2,4-triazole-1-yl)carbonyl)sulfamoyl]-5-methylthiophene-3-carboxylat (bekannt unter dem common name "Thiencarbazone-methyl").

Die herbizide Wirkung von substituierten Thien-3-yl-sulfonylaminocarbonyl-triazolinonen ist aus dem Dokument DE 199 33 260 A1 und aus Dokument WO 01/05788 A1, in welchem die Priorität der DE 199 33 260 A1 beansprucht wird, bekannt.

Eine besonders gute herbizide Wirkung haben ausgewählte substituierte Thien-3-yl-sulfonylamino-carbonyltriazolinone, wie z.B. Thiencarbazone-methyl.

Aufgrund der guten Wirkung wurde die Verbesserung der Verfahren zur Herstellung der vorgenannten Herbizide, auch im Hinblick auf die Umweltverträglichkeit der Herstellung, zu einem beständigen Bemühen der chemischen Forschung.

Dokument DE 19933260 A1 offenbart, neben vier weiteren Verfahrensalternativen, mit der Alternative (c) ein Verfahren zur Herstellung von substituierten Thien-3-yl-sulfonylaminocarbonyl-triazolinonen durch Umsetzung von Sulfonylisocyanate mit 5-methoxy-4-methyl-2,4-dihydro-3H-1,2,4-triazol-3-on. Bei dieser vorgenannten Verfahrensalternative wird die Möglichkeit der Umsetzung von substituierten Thiophen-3-sulfonsäurechloriden mit substitutierten Triazolinonen und Metall(thio)cyanaten, gegebenenfalls in Gegenwart eines Reaktionshilfsmittels, und gegebenenfalls in Gegenwart eines Verdünnungsmittels lediglich durch ein Formelschema skizziert (vgl. DE 19933260 A1, Seite 7), aber nicht durch ein ausgeführtes Beispiel belegt.

Im Zusammenhang mit den Verfahrensalternativen (a) bis (e) werden in DE 19933260 A1 optionale Reaktionshilfsmittel offenbart. Diese betreffen für derartige Umsetzungen üblicherweise einsetzbare Säurebindemittel. Die auf Seite 9 von DE 19933260 A1 genannte Auswahl, umfasst auch Kalium-tert-butylat, sowie basische Stickstoffverbindungen, wie z.B. Amine darunter auch Tributylamin und Pyridine wie z.B. 2-Methylpyridine, 3-Methylpyridin, 5-Ethyl-2-methyl-pyridin, 2,6-Dimethylpyridin.

Dagegen werden Imidazole in DE 19933260 A1 nicht als Reaktionshilfsmittel genannt.

In Dokument DE 10 2004 063192 A1 wird zur Herstellung des Sulfonylisocyanats, unter Anwendung der in DE 19933260 A1 offenbarten Verfahrensalternative (d.h. Herstellung von Thiencarbazone-methyl durch Umsetzung von Sulfonylisocyanate mit 5-methoxy-4-methyl-2,4-dihydro-3H-1,2,4-triazol-3-on) ein Verfahren ausgehend von Sulfonamid und Phosgen vorgeschlagen.

Jedoch ist das in DE 10 2004 063192 A1 offenbarte Verfahren, aufgrund der Verwendung von Phosgen, das als hochtoxisch eingestuft wird, gefährlich und somit aufwendig und teuer.

Vor diesem Hintergrund besteht die Aufgabe der Erfindung in der Bereitstellung eines verbesserten Verfahrens zur Herstellung von substituierten Thien-3yl-sulfonylamino-carbonyltriazolinonen, insbesondere von Thiencarbazone-methyl, d.h. der Verbindung der Formel (I), wobei das verbesserte Verfahren die Herstellung der Zielverbindung Thiencarbazone-methyl in einer hohen Reinheit und Ausbeute ermöglichen soll.

Gelöst wird die Aufgabe durch das Verfahren nach Anspruch 1 zur Herstellung der Verbindung der Formel (I) 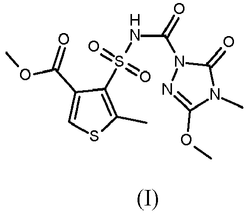 durch Umsetzung der Verbindung der Formel (II) 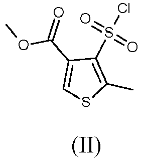 mit einem Metallcyanat der Formel (III)

MeOCN (III)

, worin Me für Li, Na, K oder, Cs steht mit der Verbindung der Formel (IV) 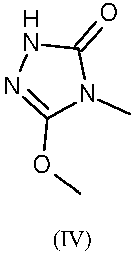 , wobei die Umsetzung in Gegenwart eines Imidazols der Formel (V) 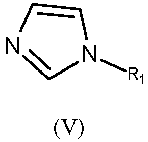 , worin der Rest R1 für ein unsubstitutiertes (C₁-C₁₂)-Alkyl oder für ein unsubstitutiertes Benzyl steht, erfolgt.

Überraschend wurde im Zusammenhang mit dem erfindungsgemäßen Verfahren gefunden, dass der Einsatz von alkylsubstitutierten Imidazolen, insbesondere von N-Alkylimidazolen die Herstellung von substituierten Thien-3yl-sulfonylaminocarbonyl-triazolinonen, insbesondere die Herstellung von Methyl 4-[(4,5-dihydro-3-methoxy-4-methyl-5-oxo-1*H*-1,2,4-triazole-1-yl)carbonyl)sulfamoyl]-5-methylthiophene-3-carboxylat(Thiencarbazone-methyl) in einer besonders hohen Reinheit und Ausbeute ermöglicht.

Der Erfindung liegt somit die Erkenntnis zugrunde, dass sich die Verbindung der Formel (I) Thiencarbazone-methyl unter bestimmten Bedingungen, nämlich in Gegenwart einer in 1-Position substituierten Imidazol-Base oder in Gegenwart einer Basen-Mischung, welche eine in 1-Position substituierte Imidazol-Base (N-Alkylimidazol) enthält, direkt aus 4-Methoxycarbonyl-2-methyl-thiophen-3-sulfonsäurechlorid (Verbindung der Formel (II)) mit einer hohen Reinheit und Ausbeute herstellbar ist.

Reaktionsschema:

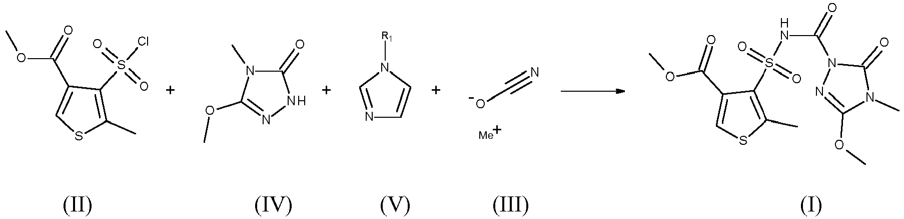

Das vorstehende Reaktionsschema zeigt, dass bei Durchführung der Reaktion als Eintopfsynthese zu Reaktionsbeginn ein 4-Komponenten-System vorliegt.

Im Fall einer zweistufigen Reaktionsführung wird das Edukt 5-methoxy-4-methyl-2,4-dihydro-3H-1,2,4-triazol-3-on der Formel (IV) erst nach Bildung des Sulfonylisocyanats aus Verbindungen der Formeln (II) und (III) in Gegenwart eines N-Alkylimidazols der Formel (V) zu dem Gemisch zugegeben.

Die Synthese der Edukte, d.h. die Synthese der Verbindungen der Formel (II) und (IV) im vorgenannten Reaktionsschema, ist in DE 19933260 A1 offenbart. Die Verbindungen der Formel (III) und (V) sind kommerziell erhältlich.

Es wird vermutet, dass die in 1-Position durch einen Alkylrest substituierten Imidazole (N-Alkylimidazole) bei der Bildung des Sulfonylisocyanats besonders gut als Aktivatoren (= Aktivierungsmittel) und/oder Stabilisatoren bei der Bildung des Sulfonylisocyanats wirken.

Der Kern der vorliegenden Erfindung betrifft jedoch die überraschende Erkenntnis, dass das Metallcyanat mit der Sulfonylgruppe der jeweiligen Thiophenverbindung (die im vorstehenden Schema mit dem Methyl-4-(chlorsulfonyl)-5-methylthiophen-3-carboxylate der Formel (II)) in Gegenwart eines N-Alkylimidazols der Formel (V) selektiv zu einem Sulfonylisocyanat reagiert, und dass sich aus diesem anschließend das substituierte Thien-3yl-sulfonylamino-carbonyltriazolinon (im vorstehenden Reaktionsschema die Zielverbindung Thiencarbazone-methyl (I)) bildet.

Die unerwartete Selektivität der im Schema dargestellten Reaktion besteht darin, dass das Triazolinon (IV) erst nach Bildung des Sulfonylisocyanats mit demselben weiter zu Thiencarbazone-methyl, reagiert.

Außerdem ist im Hinblick auf den Einsatz eines Metallcyanats bei einer chemischen Reaktion ganz allgemein zu bedenken, dass Metallcyanate starke Basen sind (Natriumcyanat (NaOCN) hat z.B. einen pH von 10). Zugleich wirken Metallcyanate als O-Nukleophile.

In Gegenwart eines N-Alkylimidazols, insbesondere in Gegenwart von N-Methylimidazol, wirkt sich die O-Nukleophilie der erfindungsgemäß eingesetzten Metallcyanate überraschenderweise aber gerade nicht auf den Verlauf der vorliegenden Reaktion, d.h. deren Ausbeute und deren Selektivität aus.

Das gilt nicht für den Einsatz anderer basischer Stickstoffverbindungen wie Pyridine, Picoline oder 4-(Dimethylamino)-pyridin (DMAP), die als klassische Aktivatoren für, zum Beispiele, Carbonsäurehalogenide anzusehen sind. Das Gleiche gilt für den Einsatz von anorganischen Basen wie K₂CO₃ oder Kalium-tert-butylat (KOtBut) die sich ebenfalls als nicht geeignet erwiesen haben.

So wurde durch Beispiel belegt, dass die Thiencarbazone-methyl-Ausbeute bei Anwendung des erfindungsgemäßen Verfahrens im Vergleich zur Durchführung der Reaktion unter Einsatz anderer alternativer basischer Stickstoffverbindungen unerwartet hoch ist.

Bevorzugt ist die Durchführung des erfindungsgemäßen Verfahrens in Gegenwart eines Imidazols der Formel (V), worin der Rest R₁ ein unsubstituiertes (C₁-C₆)-Alkyl, oder ein unsubstituiertes Benzyl ist. Die Durchführung des Verfahrens in Gegenwart eines Imidazols der Formel (V), worin der Rest R₁ ein substituiertes (C₁-C₆)-Alkyl, oder ein substituiertes Benzyl ist nicht bevorzugt, liegt jedoch ebenfalls im Rahmen der Erfindung.

Besonders bevorzugt ist die Durchführung des erfindungsgemäßen Verfahrens in Gegenwart eines Imidazols der Formel (V), worin der Rest R₁ ein unsubstituiertes (C₁-C₄)-Alkyl ist. Unverzweigte (C₁-C₄)-Alkyl, d.h. Methyl, Ethyl, n-Propyl oder n-Butyl für R₁ sind ganz besonders bevorzugt.

Am meisten bevorzugt ist die Durchführung des erfindungsgemäßen Verfahrens in Gegenwart von N-Methylimidazol (NMI), d.h. wenn. der Rest R₁ in Formel (V) für Methyl steht.

Zur Herstellung der Verbindung der Formel (I) werden bei dem erfindungsgemäßen Verfahren die Reagenzien bevorzugt äquimolar oder im Überschuss eingesetzt.

In allgemein werden je Mol Sulfonsäurechlorid, d.h. der Verbindung der Formel (II) 1 bis 2,5 Mol, bevorzugt 1 bis 2 Mol, besonders bevorzugt 1 bis 1,8 Mol des Metallcyanats der Formel MeOCN (III) eingesetzt. Aber auch der Einsatz größerer Überschüsse der Verbindung der Formel (III) liegt im Rahmen der Erfindung.

In einer bevorzugten Ausführungsform steht Me in Verbindungen der Formel MeOCN (III) für Na oder für K. Das bei dem erfindungsgemäßen Verfahren am meisten bevorzugte Metallcyanat ist NaOCN. Weiterhin werden im Allgemeinen je Mol Sulfonsäurechlorid, d.h. von der Verbindung der Formel (IV) (5-methoxy-4-methyl-2,4-dihydro-3H-1,2,4-triazol-3-on) ebenfalls 1 bis 2 Mol, bevorzugt 1 bis 1,5 Mol, besonders bevorzugt 1 bis 1,1 Mol eingesetzt.

Aber auch der Einsatz größerer Überschüsse der Verbindung der Formel (IV) liegt im Rahmen der Erfindung.

Die Durchführung des Verfahrens erfolgt in einem organischen Lösungsmittel, wobei unpolare und polare Lösungsmittel geeignet sind.

Als unpolare Lösungsmittel kommen Toluol, Chlorbenzol, Xylol, Methyl-tert-butylether, Methylisopropylether, Ethylacetat, Isopropylacetat und Butylacetat in Frage.

Besonders bevorzugte polare Lösungsmittel sind ausgewählt aus der Gruppe bestehend aus Acetonitril, Butyronitril, Tetrahydrofuran (THF), Methyl-THF, Dimethoxyethan, Sulfolan, Dimethylformamid und Dimethylacetamid.

Am meisten bevorzugt als Lösungsmittel sind Acetonitril und THF.

Auch Gemische von verschiedenen Lösungsmitteln können eingesetzt werden.

Bevorzugt erfolgt die Umsetzung der Edukte in einem Temperaturbereich von 20° bis 110° über eine Dauer (Reaktionszeit, Reaktionsdauer) von 3 Stunden bis 24 Stunden.

Ganz besonders bevorzugt ist die Umsetzung der Edukte in einem Temperaturbereich von 30° bis 90°. Am meisten bevorzugt erfolgt die Umsetzung der Edukte in einem Temperaturbereich von 50° bis 80°.

Ein weiterer Aspekt der Erfindung betrifft die Verwendung eines Imidazols der Formel (V) 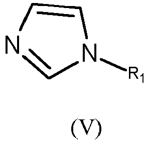 , worin der Rest R₁ für ein unsubstitutiertes (C₁-C₁₂)-Alkyl oder für ein unsubstitutiertes Benzyl steht, zur Herstellung der Verbindung der Formel (I)

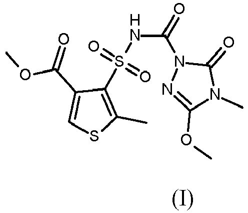

Bevorzugt ist die Verwendung eines Imidazols der Formel (V), worin R₁ ein unsubstituiertes (C₁-C₄)-Alkyl steht.

Besonders bevorzugt ist die Verwendung eines Imidazols der Formel (V), worin R₁ für ein unverzweigtes und unsubstituiertes (C₁-C₄)-Alkyl, d.h. für Methyl, Ethyl, n-Propyl oder n-Butyl steht.

Am meisten bevorzugt ist die Verwendung eines Imidazols der Formel (V), worin R₁ für Methyl steht.

### BEISPIELE:

Das erfindungsgemäße Herstellungsverfahren kann als Eintopfverfahren oder als zweistufige Reaktion durchgeführt werden.

Beim Eintopfverfahren werden alle Chemikalien in einem organischen Lösemittel gemischt und dann unter Rühren erhitzen. Um die Exothermie der Reaktion besser zu kontrollieren, ist es auch empfehlenswert, die Reagenzien der Formel (III), (IV) und (V) in z. B. Acetonitril vorzulegen, um dann das Sulfonylchlorid der Formel (II) als Lösung bei der Rektionstemperatur langsam hinzu zu dosieren.

Während der Reaktion fällt der Wirkstoff oder dessen Salz aus dem Reaktionsgemisch aus und kann einfach ab filtriert werden.

Besonders vorteilhaft ist es, am Ende der Reaktion eine anorganische Base wie z.B. LiOH, K₂CO₃, NaHCO₃, NaOH, , Na₂CO₃, CaCO₃ oder KOH zu dem Gemisch zuzusetzen, um den Wirkstoff als unlösliches Li, Na, K oder Ca-Salz zu isolieren. Bevorzugt ist die Verwendung von NaHCO₃. Auf diese Weise gelingt die Isolierung des Wirkstoffes in sehr hoher Reinheit.

Die Menge der einzusetzenden Base hängt davon ab, wieviel Metallcyanat bei Durchführung der Reaktion eingesetzt wird. Bei Einsatz von nur 1 Äquivalent (Eq) NaOCN ist dann entsprechend 1 Eq. Base notwendig, um den Wirkstoff komplett in das Salz zu überführen (Beispiel 1). Setzt man beispielsweise 2 Eq des Metallcyanats, z.B. 2 Eq NaOCN, zur Durchführung der Reaktion ein, so ist keine zusätzliche Base notwendig (vgl. Beispiel 3).

Durch Behandlung des Salzes mit einer Säure (z.B. HCl, H₂SO₄) wird der Wirkstoff freigesetzt und nach der Filtration isoliert.

Es ist auch möglich, zuerst die Verbindung der Formel (II) mit MeOCN in Gegenwart der Verbindung der Formel (V) umzusetzen, um das Sulfonylisocynat der Formel (VI) herzustellen. In einem zweiten Schritt wird dann das Sulfonylisocyanat, ggf. ohne vorherige Isolierung, mit 5-methoxy-4-methyl-2,4-dihydro-3H-1,2,4-triazol-3-on der Formel (IV) umgesetzt.

Die Analyse, Identifizierung und Gehaltsbestimmung von Sulfonylisocynat nach dem Schritt 1 basiert auf der Derivatisierung mit Methanol zum entsprechenden Carbamat der Formel (VII).

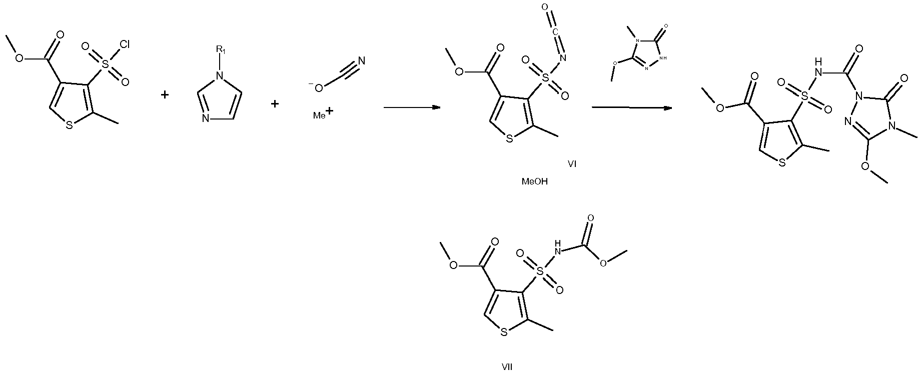

Falls notwendig, kann das Isocyanat isoliert und durch Destillation im Vakuum gereinigt werden.

**TABELLE 1**

| Tabellarischer Vergleich der Ausbeuten aufgrund LC-Analytik bei Einsatz verschiedener Reaktionshilfsmittel zur Herstellung von Methyl 4-[(4,5-dihydro-3-methoxy-4-methyl-5-oxo-1*H*-1,2,4-triazole-1-yl)carbonyl)sulfamoyl]-5-methylthiophene-3-carboxylat (Thiencarbazone-methyl), in Acetonitrile, NaOCN 2 Eq. | | | |
|---|---|---|---|
| Vergleich No. | Menge Aktivator / Mol. Eq | Zeit (Std)/T°C | Zielprodukt LC, Area % |
| 1 | 2-Methylpyridin / 1 | 12/80 | 26 |
| 2 | 3-Methylpyridin / 1 | 12/80 | 36 |
| 3 | 3-Methylpyridin / 0.1 | 12/80 | 25 |
| 4 | 2-methyl-5-ethylpyridin / 1 | 12/80 | 27 |
| 5 | 2-Methyl-5-ethylpyridin / 5 | 12/80 | 29 |
| 6 | p-Dimethylaminopyridin / 0,5 | 12/80 | 34 |
| 7 | 2,6-dimethylpyridin / 1 | 12(80) | 37 |
| 8 | Tributylamine / 1 | 12(60) | 5 |
| 9 | KOtBut / 1 | 12 (80) | 0 |
| 10 | N-Butylimidazol/1 | 12(80) | 84 |
| 11 | N-Methylimidazol / 1 | 12(80) | 92 |
| 12 | N-Methylimidazol / 0,8 | 12(80) | 91 |

Die oben stehende Tabelle belegt, dass die Thiencarbazone-methyl-Ausbeute bei Anwendung des erfindungsgemäßen Verfahrens bei Einsatz von N-Butylimidazol und N-Methylimidazol im Vergleich zu anderen Stickstoffbasen unerwartet hoch ist. Dagegen ist beim vergleichenden Einsatz der Stickstoffbase Tributylamin das durch Flüssigchromatographie (LC) nachweisbare Zielprodukt Thiencarbazone-methyl in einem niedrigen %-Bereich. Bei Einsatz des Säurebindemittels Kalium-tert-butylat (KOtBut) ist ein Produktnachweis gar nicht möglich.

### SYNTHESEBEISPIELE

### Beispiel 1

### Methyl 4-[(4,5-dihydro-3-methoxy-4-methyl-5-oxo-1H-1,2,4-triazole-1-yl)carbonyl)sulfamoyl]-5-methylthiophene-3-carboxylat (Eintopfverfahren)

25.4 g von Methyl-4-(chlorsulfonyl)-5-methylthiophen-3-carboxylat, 6,5 g NaOCN, 12,9 g N-Methylimidazol und 12,9 g 5-Methoxy-4-methyl-2,4-dihydro-3H-1,2,4-triazol-3-on wird in 200 ml Acetonitril vorgelegt und auf 70°C erhitzt. Das Gemisch wurde unter Rühren 12 Std bei dieser Temperatur erhitzt und auf 20°C abgekühlt.

Dem Gemisch wurden 8,5 g NaHCO₃ zugesetzt und die Suspension wurde 3 Std bei 20°C nachgerührt. Der Niederschlag wurde abfiltriert, mit 50 ml 10-iger % HCl und 100 ml Wasser gewaschen und bei 50°C getrocknet. Man erhielt 31,6 g des_Methyl 4-[(4,5-dihydro-3-methoxy-4-methyl-5-oxo-1*H*-1,2,4-triazole-1-yl)carbonyl)sulfamoyl]-5-methylthiophene-3-carboxylats, 80 % der Theorie mit einem Schmelzpunkt von 201°C, und einer Reinheit von 99 %.

### Beispiel 2

### Methyl 4-[(4,5-dihydro-3-methoxy-4-methyl-5-oxo-1H-1,2,4-triazole-1-yl)carbonyl)sulfamoyl]-5-methylthiophene-3-carboxylat (Eintopfverfahren)

25.4 g. von Methyl-4-(chlorsulfonyl)-5-methylthiophen-3-carboxylat, 11,7 g NaOCN, 8,2 g N-Methylimidazol und 12,9 g 5-Methoxy-4-methyl-2,4-dihydro-3H-1,2,4-triazol-3-on wird in 220 ml Acetonitril vorgelegt und auf 70°C erhitzt. Das Gemisch wurde unter Rühren 18 Std bei dieser Temperatur erhitzt und auf 20°C abgekühlt. Dem Gemisch wurden 1,7 g NaHCO₃ zugesetzt und die Suspension wurde 2 Std bei 20°C nachgerührt. Das Reaktionsgemisch wurde auf 60°C erhitzt, der Niederschlag wurde ab filtriert und mit 50 ml Acetonitril gewaschen. Danach wurde der Niederschlag mit 70 ml 20-iger % H₂SO₄, 100 ml heißem (70°C) Wasser und 50 ml Acetone gewaschen und bei 50°C getrocknet. Man erhielt 31,4 g des Methyl 4-[(4,5-dihydro-3-methoxy-4-methyl-5-oxo-1*H*-1,2,4-triazole-1-yl)carbonyl)sulfamoyl]-5-methylthiophene-3-carboxylats, 79 % der Theorie mit einer Reinheit von 98 %.

### Beispiel 3

### Methyl 4-[(4,5-dihydro-3-methoxy-4-methyl-5-oxo-1H-1,2,4-triazole-1-yl)carbonyl)sulfamoyl]-5-methylthiophene-3-carboxylat (Eintopfverfahren)

25.4 g von Methyl-4-(chlorsulfonyl)-5-methylthiophen-3-carboxylat, 13 g NaOCN, 12,9 g N-Methylimidazol und 12,9 g 5-Methoxy-4-methyl-2,4-dihydro-3H-1,2,4-triazol-3-on wird in 200 ml Acetonitril vorgelegt und auf 70°C erhitzt. Das Gemisch wurde unter Rühren 12 Std bei dieser Temperatur erhitzt und auf 20°C abgekühlt.

Der Niederschlag wurde abfiltriert, mit 50 ml 10-iger % HCl und 100 ml Wasser gewaschen und bei 50°C getrocknet. Man erhielt 33,6 g des Methyl 4-[(4,5-dihydro-3-methoxy-4-methyl-5-oxo-1*H*-1,2,4-triazole-1-yl)carbonyl)sulfamoyl]-5-methylthiophene-3-carboxylats, 84,4 % der Theorie mit einer Reinheit von 98 %.

### Beispiel 4

### Methyl 4-[(4,5-dihydro-3-methoxy-4-methyl-5-oxo-1H-1,2,4-triazole-1-yl)carbonyl)sulfamoyl]-5-methylthiophene-3-carboxylat (Zweistufen-Verfahren)

25.4 g von Methyl-4-(chlorsulfonyl)-5-methylthiophen-3-carboxylate, 6,5 g NaOCN, 12,9 g N-Methylimidazol wurden in 150 ml Acetonitril vorgelegt und das Gemisch 4 Std bei 50°C erhitzt. Die Suspension wurde unter Argon über eine Glassfritte filtriert und dem Filtrat 12.5 g 5-Methoxy-4-methyl-2,4-dihydro-3H-1,2,4-triazol-3-on zugegeben. Anschließend wurde das Gemisch 8 Std bei 70°C erhitzt und auf 20°C abgekühlt.

Dem Gemisch wurden 8 g NaHCO₃ und 1 ml Wasser zugesetzt und die Suspension wurde 3 Std nachgerührt. Der Niederschlag wurde abfiltriert, mit 50 ml 10 %-iger HCl und 100 ml Wasser gewaschen und bei 50°C getrocknet. Man erhielt 29,6 g des Methyl 4-[(4,5-dihydro-3-methoxy-4-methyl-5-oxo-1*H*-1,2,4-triazole-1-yl)carbonyl)sulfamoyl]-5-methylthiophene-3-carboxylats 75,6 % der Theorie mit einem Schmelzpunkt 201°C und einer Reinheit von >99 %.

### Beispiel 5

### Methyl 4-[(4,5-dihydro-3-methoxy-4-methyl-5-oxo-1H-1,2,4-triazole-1-yl)carbonyl)sulfamoyl]-5-methylthiophene-3-carboxylat (Zweistufen-Verfahren)

25.4 g von Methyl-4-(chlorsulfonyl)-5-methylthiophen-3-carboxylate, 11,7 g NaOCN, 12,3 g N-Methylimidazol wurden in 150 ml Acetonitril vorgelegt und das Gemisch 4 Std bei 50°C erhitzt. Die Suspension wurde unter Argon über eine Glassfritte filtriert und dem Filtrat 12.5 g 5-Methoxy-4-methyl-2,4-dihydro-3H-1,2,4-triazol-3-on zugegeben. Anschließend wurde das Gemisch 12 Std bei 70°C erhitzt und auf 20°C abgekühlt.

Dem Gemisch wurden 1.7 g NaHCO₃ und 1 ml Wasser zugesetzt und die Suspension wurde 3 Std nachgerührt. Der Niederschlag wurde abfiltriert, mit 50 ml 10 %-iger HCl und 100 ml heißem Wasser gewaschen und bei 50°C getrocknet. Man erhielt 30,5 g des Methyl 4-[(4,5-dihydro-3-methoxy-4-methyl-5-oxo-1*H*-1,2,4-triazole-1-yl)carbonyl)sulfamoyl]-5-methylthiophene-3-carboxylats 77 % der Theorie mit einer Reinheit von 98 %.

### Beispiel 6

### Methyl 4-(methoxycarbonylsulfamoyl)-5-methyl-thiophen-3-carboxylat

25.4 g von Methyl-4-(chlorsulfonyl)-5-methylthiophene-3-carboxylat, 6,5 g NaOCN, 12,9 g N-Methylimidazol wurden in 150 ml Acetonitril vorgelegt und das Gemisch 4 Std bei 50°C erhitzt. Die Suspension wurde unter Argon über eine Glassfritte filtriert und mit 30 ml Methanol versetzt. Nach 1 Std wurde die Lösung komplett eingeengt und der Niederschlag mit Waser gewaschen und getrocknet. Man erhielt 24 g Methyl 4-(methoxycarbonylsulfamoyl)-5-methyl-thiophen-3-carboxylat (¹H NMR: (d⁶ DMSO) 2,72 (s), 3,62 (s), 3,79 (s), 8.01 (s), 12.01 (s) ppm).

## Patentansprüche

1. Verfahren zur Herstellung der Verbindung der Formel (I) 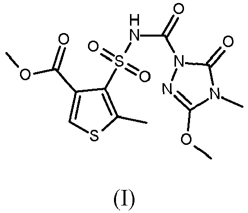 durch Umsetzung der Verbindung der Formel (II) 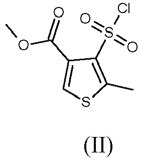 mit einem Metallcyanat der Formel (III)
MeOCN (III)
, worin Me für Li, Na, K oder Cs steht
und
mit der Verbindung der Formel (IV) 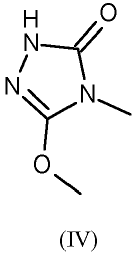 , wobei die Umsetzung in Gegenwart eines Imidazols der Formel (V) 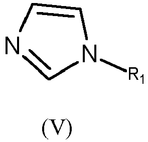 , worin der Rest R₁ für ein unsubstitutiertes (C₁-C₁₂)-Alkyl oder für ein unsubstitutiertes Benzyl steht, erfolgt.

2. Verfahren zur Herstellung von Verbindungen der Formel (I) nach Anspruch 1, **dadurch gekennzeichnet, dass** R₁ ein unsubstituiertes (C₁-C₆)-Alkyl oder ein unsubstituiertes Benzyl ist.

3. Verfahren zur Herstellung von Verbindungen der Formel (I) nach Anspruch 2, **dadurch gekennzeichnet, dass** R₁ ein unsubstituiertes (C₁-C₄)-Alkyl ist.

4. Verfahren zur Herstellung von Verbindungen der Formel (I) nach Anspruch 3, **dadurch gekennzeichnet, dass** R₁ für Methyl, Ethyl, n-Propyl oder n-Butyl steht.

5. Verfahren zur Herstellung von Verbindungen der Formel (I) nach Anspruch 4, **dadurch gekennzeichnet, dass** R₁ für Methyl steht.

6. Verfahren zur Herstellung von Verbindungen der Formel (I) nach einem der Ansprüche 1 bis 5, **dadurch gekennzeichnet, dass** Me in Verbindungen der Formel MeOCN (III) für Na oder für K steht.

7. Verfahren zur Herstellung von Verbindungen der Formel (I) nach einem der Ansprüche 1 bis 6, **dadurch gekennzeichnet, dass** die Umsetzung in einem polaren Lösungsmittel ausgewählt aus der Gruppe bestehend aus Acetonitril, Butyronitril, Tetrahydrofuran (THF), Methyl-THF, Dimethoxyethan, Sulfolan, Dimethylformamid und Dimethylacetamid erfolgt.

8. Verfahren zur Herstellung von Verbindungen der Formel (I) nach einem der Ansprüche 1 bis 6, **dadurch gekennzeichnet, dass** die Umsetzung in einer Lösungsmittelmischung bestehend aus Acetonitril und THF erfolgt.

9. Verfahren zur Herstellung von Verbindungen der Formel (I) nach einem der Ansprüche 1 bis 8, **dadurch gekennzeichnet, dass** die Umsetzung der Edukte
- in einem Temperaturbereich von 20° bis 110° und
- in Reaktionszeit von 3 Stunden bis 24 Stunden
erfolgt.

10. Verfahren zur Herstellung von Verbindungen der Formel (I) nach Anspruch 9, **dadurch gekennzeichnet, dass** die Umsetzung der Edukte in einem Temperaturbereich von 30° bis 90° erfolgt.

11. Verfahren zur Herstellung von Verbindungen der Formel (I) nach Anspruch 10, **dadurch gekennzeichnet, dass** die Umsetzung der Edukte in einem Temperaturbereich von 50° bis 80° erfolgt.

12. Verwendung eines Imidazols der Formel (V) 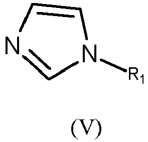 , worin der Rest R₁ für ein unsubstitutiertes (C₁-C₁₂)-Alkyl oder für ein unsubstitutiertes Benzyl steht, für ein Verfahren zur Herstellung der Verbindung der Formel (I) 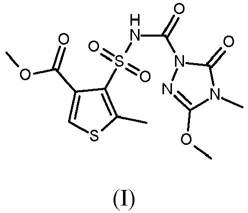 gemäß einem der Ansprüche 1 bis 9.

13. Verwendung eines Imidazols der Formel (V) nach Anspruch 12, **dadurch gekennzeichnet, dass** R₁ ein unsubstituiertes (C₁-C₄)-Alkyl steht.

14. Verwendung eines Imidazols der Formel (V) nach Anspruch 13, **dadurch gekennzeichnet, dass** R₁ für Methyl, Ethyl, n-Propyl oder n-Butyl steht.

15. Verwendung eines Imidazols der Formel (V) nach Anspruch 14, **dadurch gekennzeichnet, dass** R₁ für Methyl steht.

## Claims

1. Method for preparing the compound of the formula (I) 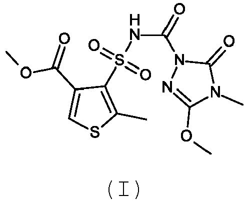 by reacting the compound of the formula (II) 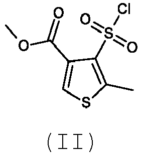 with a metal cyanate of the formula (III)
MeOCN (III)
where Me is Li, Na, K or Cs
and
with the compound of the formula (IV) 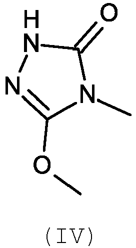 wherein the reaction is carried out in the presence of an imidazole of the formula (V) 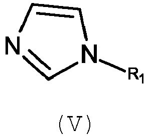 where the radical R₁ is an unsubstituted (C₁-C₁₂) -alkyl or an unsubstituted benzyl.

2. Method for preparing compounds of the formula (I) according to Claim 1, **characterized in that** R₁ is an unsubstituted (C₁-C₆)-alkyl or an unsubstituted benzyl.

3. Method for preparing compounds of the formula (I) according to Claim 2, **characterized in that** R₁ is an unsubstituted (C₁-C₄)-alkyl.

4. Method for preparing compounds of the formula (I) according to Claim 3, **characterized in that** R₁ is methyl, ethyl, n-propyl or n-butyl.

5. Method for preparing compounds of the formula (I) according to Claim 4, **characterized in that** R₁ is methyl.

6. Method for preparing compounds of the formula (I) according to any of Claims 1 to 5, **characterized in that** Me in compounds of the formula MeOCN (III) is Na or K.

7. Method for preparing compounds of the formula (I) according to any of Claims 1 to 6, **characterized in that** the reaction is carried out in a polar solvent selected from the group consisting of acetonitrile, butyronitrile, tetrahydrofuran (THF), methyl-THF, dimethoxyethane, sulfolane, dimethylformamide and dimethylacetamide.

8. Method for preparing compounds of the formula (I) according to any of Claims 1 to 6, **characterized in that** the reaction is carried out in a solvent mixture consisting of acetonitrile and THF.

9. Method for preparing compounds of the formula (I) according to any of Claims 1 to 8, **characterized in that** the reaction of the reactants is conducted
- in a temperature range of 20° to 110° and
- in a reaction time of 3 hours to 24 hours.

10. Method for preparing compounds of the formula (I) according to Claim 9, **characterized in that** the reaction of the reactants is conducted in a temperature range of 30° to 90°.

11. Method for preparing compounds of the formula (I) according to Claim 10, **characterized in that** the reaction of the reactants is conducted in a temperature range of 50° to 80°.

12. Use of an imidazole of the formula (V) 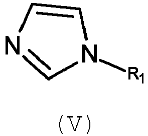 where the radical R₁ is an unsubstituted (C₁-C₁₂) -alkyl or an unsubstituted benzyl, for a method for preparing the compound of the formula (I) 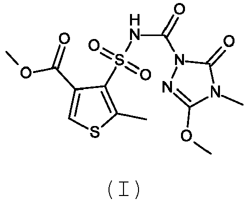 according to any of Claims 1 to 9.

13. Use of an imidazole of the formula (V) according to Claim 12, **characterized in that** R₁ is an unsubstituted (C₁-C₄)-alkyl.

14. Use of an imidazole of the formula (V) according to Claim 13, **characterized in that** R₁ is methyl, ethyl, n-propyl or n-butyl.

15. Use of an imidazole of the formula (V) according to Claim 14, **characterized in that** R₁ is methyl.

## Revendications

1. Procédé pour la préparation du composé de formule (I) 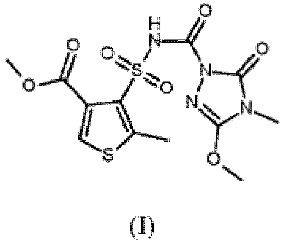 par transformation du composé de formule (II) 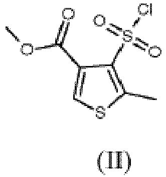 avec un cyanate métallique de formule (III)
MeOCN (III),
dans laquelle Me représente Li, Na, K ou Cs et
avec le composé de formule (IV) 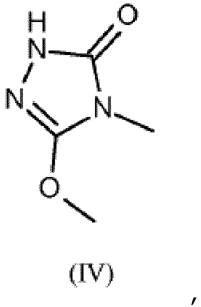 la transformation étant réalisée en présence d'un imidazole de formule (V) 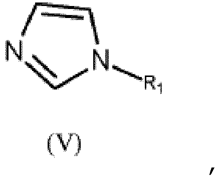 dans laquelle le radical R₁ représente un (C₁-C₁₂)-alkyle non substitué ou un benzyle non substitué.

2. Procédé pour la préparation de composés de formule (I) selon la revendication 1, **caractérisé en ce que** R₁ est un (C₁-C₆)-alkyle non substitué ou un benzyle non substitué.

3. Procédé pour la préparation de composés de formule (I) selon la revendication 2, **caractérisé en ce que** R₁ est un (C₁-C₄)-alkyle non substitué.

4. Procédé pour la préparation de composés de formule (I) selon la revendication 3, **caractérisé en ce que** R₁ représente méthyle, éthyle, n-propyle ou n-butyle.

5. Procédé pour la préparation de composés de formule (I) selon la revendication 4, **caractérisé en ce que** R₁ représente méthyle.

6. Procédé pour la préparation de composés de formule (I) selon l'une quelconque des revendications 1 à 5, **caractérisé en ce que** Me dans les composés de formule MeOCN (III) représente Na ou K.

7. Procédé pour la préparation de composés de formule (I) selon l'une quelconque des revendications 1 à 6, **caractérisé en ce que** la transformation est réalisée dans un solvant polaire choisi dans le groupe constitué par l'acétonitrile, le butyronitrile, le tétrahydrofuranne (THF), le méthyl-THF, le diméthoxyéthane, le sulfolane, le diméthylformamide et le diméthylacétamide.

8. Procédé pour la préparation de composés de formule (I) selon l'une quelconque des revendications 1 à 6, **caractérisé en ce que** la transformation est réalisée dans un mélange de solvants constitué par l'acétonitrile et le THF.

9. Procédé pour la préparation de composés de formule (I) selon l'une quelconque des revendications 1 à 8, **caractérisé en ce que** la transformation des éduits est réalisée
- dans une plage de température de 20° à 110° et
- en un temps de réaction de 3 heures à 24 heures.

10. Procédé pour la préparation de composés de formule (I) selon la revendication 9, **caractérisé en ce que** la transformation des éduits est réalisée dans une plage de température de 30° à 90°.

11. Procédé pour la préparation de composés de formule (I) selon la revendication 10, **caractérisé en ce que** la transformation des éduits est réalisée dans une plage de température de 50° à 80°.

12. Utilisation d'un imidazole de formule (V) 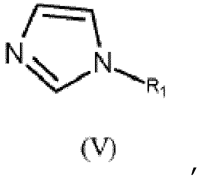 dans laquelle le radical R₁ représente un (C₁-C₁₂)-alkyle non substitué ou un benzyle non substitué, pour un procédé pour la préparation du composé de formule (I) 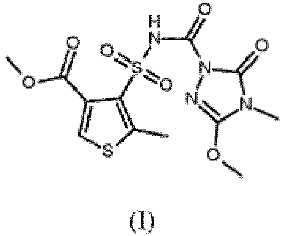 selon l'une quelconque des revendications 1 à 9.

13. Utilisation d'un imidazole de formule (V) selon la revendication 12, **caractérisée en ce que** R₁ représente un (C₁-C₄)-alkyle non substitué.

14. Utilisation d'un imidazole de formule (V) selon la revendication 13, **caractérisée en ce que** R₁ représente méthyle, éthyle, n-propyle ou n-butyle.

15. Utilisation d'un imidazole de formule (V) selon la revendication 14, **caractérisée en ce que** R₁ représente méthyle.
